# EUROPÄISCHE PATENTSCHRIFT

(11) **EP 4 221 663 B1**
(45) Veröffentlichungstag und Bekanntmachung des Hinweises auf die Patenterteilung: **15.04.2026**
(21) Anmeldenummer: 21770168.9
(22) Anmeldetag: 27.08.2021
(51) Int. Cl.: A61K 8/02, A61K 8/04, A61K 8/34, A61K 8/81, A61Q 1/14

(54) **VERBESSERTE REINIGUNGSTÜCHER GETRÄNKT MIT TRÄNKUNGSMITTELN BASIEREND AUF HYDRODISPERSIONEN**
IMPROVED CLEANING CLOTHS IMPREGNATED WITH IMPREGNATION AGENTS BASED ON HYDRODISPERSIONS
LINGETTES NETTOYANTES AMÉLIORÉES IMPRÉGNÉES D'AGENTS D'IMPRÉGNATION À BASE D'HYDRODISPERSIONS

(30) Priorität: 29.09.2020 DE 102020212221
(43) Veröffentlichungstag der Anmeldung: 09.08.2023
(73) Patentinhaber: Beiersdorf AG, 22529 Hamburg (DE)
(72) Erfinder: SIEKRO, Magalie, 25469 Halstenbek (DE); JOHNS, Nicole, 22958 Kuddewörde (DE)
(74) Vertreter: Beiersdorf AG
(86) Internationale Anmeldenummer: PCT/EP2021/073755
(87) Internationale Veröffentlichungsnummer: WO 2022/069123

(56) Entgegenhaltungen:
- EP-A1- 3 650 082
- DE-A1- 102012 200 383
- DE-A1- 102017 209 649
- DE-A1- 102018 220 913
- DATABASE GNPD [online] MINTEL; 20 December 2012 (2012-12-20), ANONYMOUS: "Facial Cleansing Wipes", XP055869559, retrieved from https://www.gnpd.com/sinatra/recordpage/1947922/ Database accession no. 1947922

## Beschreibung

Die vorliegende Erfindung betrifft ein Reinigungstuch getränkt mit einem Tränkungsmittel basierend auf einer Hydrodispersion.

Reinigungstücher sind eine besondere Form von Reinigungsmitteln, denn die Reinigungsleistung beruht auf einem Zusammenwirken von Tuch und Tränkungsmittel.

Reinigungstücher zeichnen sich dadurch aus, dass es sich um ein Reinigungsmittel handelt, das sofort einsatzbereit ist. Es ist kein zusätzliches Lösungsmittel, insbesondere Wasser erforderlich, um die Wirkung zu entfalten. Auch wird kein Lösungsmittel, insbesondere Wasser benötigt, um Verschmutzungen abzuwaschen. Reinigungstücher können bequem unterwegs und auf Reisen verwendet werden oder auch einfach einmal zwischendurch.

Die genannten Vorteile bewirken, dass die Reinigungstücher vielfältig zum Einsatz kommen, beispielsweise in der Leder- und Bodenpflege, im Hygienebereich, in der Intim- und Körperpflege. Im Zusammenhang mit der Körperpflege werden Reinigungstücher auch vorteilhaft zur Babypflege eingesetzt, für die Gesichts- und Körperreinigung, für die Reinigung der Haut im Windelbereich und als Toilettenpapier, um bei kleinen Kindern das Toilettentraining zu fördern.

Ein Gebiet, auf dem sich der Einsatz von Reinigungstüchern besonders großer Beliebtheit erfreut, ist die Gesichtsreinigung. Bei der Gesichtsreinigung müssen Verschmutzungen, die von außen auf die Haut gelangt sind, abgestorbene Zellen und überschüssiger Hauttalg entfernt werden. Zu den Verschmutzungen, die von außen auf die Haut gelangt sind, gehört auch Make-up, das wieder entfernt werden muss.

Im Bereich der dekorativen Kosmetik finden heutzutage eine Vielzahl von unterschiedlichen Stoffen Anwendung. Als Farbstoffe werden neben anorganischen Pigmenten wie Silikaten [Magnesiumsilikat (Talkum), Aluminiumsilikat (Kaolin)] und Metalloxiden (Chrom-, Eisen-, Mangan-, Titan- und Zinkoxiden) auch organische Farbpigmente eingesetzt. Als Bindemittel finden unter anderem Stearinsäureester, Lanolinalkohol und -acetat Verwendung. In vielen Formulierungen werden Wachse wie Bienenwachs oder Carnaubawachs und Öle wie Paraffinöle, Silikonöle oder Ricinusöl eingesetzt. Des Weiteren können dekorative Kosmetika Konservierungsstoffe, Antioxidantien, Verdickungsmittel und andere Zusätze enthalten.

Um diese Vielzahl sehr unterschiedlicher Stoffe von der Haut zu entfernen, bedarf es entsprechender kosmetischer Reinigungsmittel. Als gut geeignet für diesen Zweck haben sich Reinigungstücher erwiesen, weil mit der Wischbewegung der Tücher die Reinigungswirkung der im Tränkungsmittel enthaltenden oberflächenaktiven Substanzen und/oder Öle unterstützt wird.

Reinigungstücher sind an sich bekannt. Im Stand der Technik gibt es eine Vielzahl von Dokumenten, die verschiedenartige Reinigungstücher mit unterschiedlichen Tränkungsmitteln offenbaren.

Zum Beispiel ist ein kommerziell erhältliches Produkt "pure & natural Facial Cleansing Wipes" der Firma Beiersdorf bekannt (DATENBANK GNPD [Online] MINTEL; 20. Dezember 2012; Zugangsnummer 1947922). Es handelt sich um biologisch abbaubare Reinigungstücher zur Make-Up und Mascara Entfernung die aus 95% Material natürlichen Ursprungs bestehen.

DE 10157543 offenbart Emulsions-getränkte Tücher, wobei die Emulsion Chitosan und/oder Lecithin enthält. Dadurch kann der Gehalt an Emulgatoren und Konservierungsmitteln reduziert werden.

DE 10219638 beschreibt ebene, gelochte Reinigungstücher, die mit einem emulsions-artigen Tränkungsmittel getränkt sind. Diese Tücher bewirken eine sanfte Reinigung, insbesondere Gesichtsreinigung.

Im Dokument EP 1496853 werden Reinigungstücher beschrieben, die mit einem Tränkungsmittel getränkt sind, das Stärke-haltige Verbindungen enthält. Die Einarbeitung dieser Stärke-haltigen Verbindungen führt zu einem besonders angenehmen, glatten, samtigen Hautgefühl.

EP 1405632 offenbart die Einarbeitung von Zinkoxidpartikeln einer bestimmten Größe in die Tränkungsmittel und die Möglichkeit diese Tränkungsmittel auf die Tücher aufzubringen.

Im Dokument DE 102012200383 werden Reinigungstücher offenbart, die eine raue Oberfläche aufweisen. Dies führt zu einem Peelingeffekt. Um die Stärke des Peelingeffekts steuern zu können, werden verschiedene Mengen des Tränkungsmittels aufgebracht.

Im Dokument DE 202004007851 werden gefärbte Reinigungstücher und Tränkungsmittel beschrieben. Die eingesetzten Farbstoffe und Tränkungsmittel sind so gewählt, dass die Farbe auf dem Tuch verbleibt und nicht in das Tränkungsmittel übertritt.

Lipidhaltige Zusammensetzungen zur Tränkung von Tüchern werden im Dokument WO 2005/044220 A1 offenbart. Diese Tränkungsmittel bewirken, dass Reinigungsartikel getränkt mit diesen Mitteln neben einem hohen Reinigungsvermögen auch ein hohes Rückfettungsvermögen aufweisen.

Klassische Reinigungstücher können also mit Tränkungsmitteln getränkt sein, die auf die Einarbeitung "klassischer" Tenside, wie beispielsweise Alkylethersulfate, die in der Regel in flüssigen Reinigungszubereitungen eingesetzt werden, verzichten. Dies kann dazu führen, dass die Reinigungsleistung gemindert ist. Öl-haltige Reinigungstücher werden häufig zur Entfernung von wasserfesten Make-up-Zubereitungen eingesetzt. Diese Öl-haltigen Reinigungstücher haben den Nachteil, dass Öle aufgrund ihrer unterschiedlich hohen Spreitfähigkeit ins Auge gelangen können. Dies kann zu einer verminderten Verträglichkeit dieser Produkte führen. Des Weiteren bleibt bei der Verwendung Öl-haltiger Reinigungstücher oft ein öliger Rückstand auf der Haut zurück, der von vielen Anwendern als unangenehm empfunden wird.

Im StdT sind weiterhin Reinigungstücher offenbart, die eine gute Reinigungsleistung und eine gute Verträglichkeit haben. Als Beispiel sei das Dokument DE 102015212822 A1 genannt. Das Tränkungsmittel weist Mizellen auf. Das Tränkungsmittel gemäß DE 102015212822 A1 zeichnet sich dadurch aus, dass eine Mischung aus verschiedenen Tensiden enthalten ist, anionische Tenside in Form von Natrium Acylglutamaten, nichtionische Tenside in Form von Blockpolymeren und Alkylpolyglykosiden.

Reinigungstücher sollen, wie der Name impliziert, zur Reinigung verwendet werden, bevorzugt zur Reinigung der menschlichen Haut und Haare.

Neben der Grundfunktion der Reinigung gibt es mannigfaltige Verbraucherwünsche, denen bei der Weiterentwicklung von Reinigungstüchern Rechnung getragen werden soll.

So ist der Aspekt einer verbesserten Verträglichkeit ein wichtiger Aspekt. In diesem Zusammenhang sei beispielsweise erwähnt, dass bei Verbraucherumfragen wiederholt der Wunsch geäußert wird, auf Parabene zu verzichten. Gleichwohl wird hier darauf hingewiesen, dass ausgewählte Parabene in bestimmten Mengen gemäß der Verordnung (EG) Nr. 1223/2009 über kosmetische Mittel in kosmetischen Produkten zur Konservierung eingesetzt werden dürfen, weil sie als unbedenklich eingestuft werden.

Ebenso ist der Verzicht auf Silikon-Verbindungen, ein Wunsch, der von Verbrauchern häufig geäußert wird.

Darüber hinaus ist es wünschenswert, wenn die Inhaltsstoffe des Tränkungsmittels und das Tuchmaterial so weit wie möglich biologisch abbaubar sind.

Zusammenfassend sollen also Reinigungstücher bereitgestellt werden, die den Verbraucherwünschen vermehrter Verträglichkeit und verbesserter biologischer Abbaubarkeit, insbesondere des Tuchmaterials, Rechnung tragen. Gleichwohl soll aber die Reinigungsleistung derjenigen herkömmlicher Tücher entsprechen.

Gelöst werden alle diese Aufgaben durch ein Reinigungsmittel bestehend aus einem Tuch und einem Tränkungsmittel gemäß Anspruch 1. Die vorliegende Erfindung stellt daher bereit ein Reinigungsmittel bestehend aus einem Tuch, dadurch gekennzeichnet, dass das Tuch aus Fasern besteht, die natürlichen Ursprungs und biologisch abbaubar sind und einem Tränkungsmittel, dadurch charakterisiert, dass das Tränkungsmittel in Form einer Hydrodispersion vorliegt, die Silikon-frei und Paraben-frei ist, das heißt weniger als 0,001 Gewichts-%, bezogen auf das Gesamtgewicht des Tränkungsmediums enthält, enthaltend
- Phenoxyethanol,
- wenigstens zwei Substanzen, die gleichzeitig pflegend wirken und die Konservierung unterstützen und
- wenigstens zwei polymere Verbindungen, wobei wenigstens eine polymere Verbindung stabilisierend und wenigstens eine polymere Verbindung filmbildend wirkt oder wobei wenigstens eine polymere Verbindung stabilisierend und filmbildend wirkt und wenigstens eine weitere polymere Verbindung enthalten ist,
- dadurch gekennzeichnet, dass die wenigstens zwei Substanzen, die gleichzeitig pflegend wirken und die Konservierung unterstützen, ausgewählt werden aus der Gruppe Ethylhexylglycerin, Hydroxyacetophenone und Decylene Glycol.

Das Reinigungsmittel ist insbesondere auch für die Reinigung der Gesichtshaut geeignet sein und dort wiederum weiter insbesondere für den empfindlichen Augenbereich. Es handelt sich bei dem genannten Reinigungsmittel um ein kosmetisches Produkt.

Das Tränkungsmittel enthält Wasser; das Tränkungsmittel ist also ein wässriges Tränkungsmittel. Der Wassergehalt beträgt vorteilhaft 80 bis 99 Gew.-%, besonders vorteilhaft 93 bis 98,9 Gew.-%, bezogen auf das Gesamtgewicht des Tränkungsmittels.

Die Konservierung des Tränkungsmittels und somit auch des Reinigungsproduktes erfolgt durch Phenoxyethanol. Dieses Konservierungsmittel kann durch folgende Formel dargestellt werden: 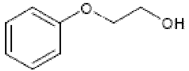 Phenoxyethanol ist vor allem in saurem und neutralem, aber auch im alkalischen Milieu wirksam und völlig ungiftig. Bereits durch den Einsatz von niedrigen Konzentrationen kann eine ausreichende Konservierung kosmetischer Produkte erreicht werden. Aufgrund seines neutralen Geschmacks fand Phenoxyethanol schnell Eingang in die pharmazeutische und kosmetische Industrie. Seine Wirkung richtet sich gegen Gram-positive und Gram-negative Bakterien, wie zum Beispiel Escherichia coli, Pseudomonas aeruginosa, Proteus mirabilis, Staphylococcus aureus sowie gegen Hefepilze, wie Candida albicans und andere Mikroorganismen. Phenoxyethanol ist eine viskose Flüssigkeit von schwachem, leicht angenehmem Geruch und einem zusammenziehenden Geschmack. Es wurde in der Natur nachgewiesen in tropischen Früchten, in Cichorium endivia sowie in grünem Tee (Camellia sinesis). Es hat einen milden, rosenähnlichen

Duft und wird für Parfümkompositionen auch als Fixatur eingesetzt. Es ist mischbar mit Aceton, Ethylalkohol und Glycerin, löslich in und Fetten, z.B. Oliven- und Erdnussöl, jedoch wenig löslich in Wasser.

Im erfindungsgemäßen Tränkungsmittel ist Phenoxyethanol mit einem Gehalt von 0,5 bis 0,98 Gew.-%, bevorzugt 0,8 bis 0,95 Gew.-% enthalten, bezogen auf das Gesamtgewicht des Tränkungsmittels.

Im erfindungsgemäßen Tränkungsmittel sind wenigstens zwei Substanzen, die gleichzeitig pflegend wirken und die Konservierung unterstützen, enthalten. Diese Substanzen sind nicht in der Liste der zugelassenen Konservierungsstoffe (Kosmetikverordnung Anlage 6; Verordnung (EG) Nr. 1223/2009, Anhang V) aufgeführt, gleichwohl haben sie aber eine stabilisierende Wirkung auf die jeweiligen kosmetischen Zubereitungen und im gemeinsamen Einsatz mit Konservierungsmitteln fördern sie die Stabilität der jeweiligen Zubereitungen. Dieses Phänomen kann also als Konservierungs-unterstützend beschrieben werden. Derartige Substanzen weisen Hydroxylgruppen auf, die bewirken, dass sich Hydrathüllen um die Moleküle bilden können. Auf diese Weise wirken sie Feuchtigkeit-spendend und somit Haut-pflegend. Im erfindungsgemäßen Tränkungsmittel sind die wenigstens zwei Substanzen, die gleichzeitig pflegend wirken und die Konservierung unterstützen, mit einem Gesamtgehalt von 0,15 bis 0,6 Gew.-%, bevorzugt 0,3 bis 0,45 Gew.-% enthalten, bezogen auf das Gesamtgewicht des Tränkungsmittels.

Erfindungsgemäß sind Kombinationen aus Phenoxyethanol und wenigstens zwei Substanzen ausgewählt aus Ethylhexylglycerin, Hydroxyacetophenone und Decylene Glycol, weiter bevorzugt ist die Kombination von Phenoxyethanol, mit den Substanzen die gleichzeitig pflegend wirken und die Konservierung unterstützen Ethylhexylglycerin und Decylene Glycol.

Das erfindungsgemäße Tränkungsmittel kann Parfüm enthalten. Das eingesetzte Parfüm ist vorteilhaft eine Mischung aus Parfüm rohstoffen. Das Parfüm ist hauptsächlich enthalten, um dem Tränkungsmittel und nach dem Tränkungsvorgang auch dem gesamten Produkt einen angenehmen Geruch zu verleihen. Weiterhin ist von ausgewählten Parfümrohstoffen auch bekannt, dass sie eine antimikrobielle Wirkung entfalten können oder aber auch die Wirkung von herkömmlichen Konservierungsmitteln unterstützen können.

Wenn in dem erfindungsgemäßen Tränkungsmittel Parfüm enthalten ist, so liegt das Parfüm mit einem Gehalt von 0,05 bis 1,0 Gew.-%, insbesondere 0,14 bis 0,4 Gew.-%, bezogen auf das Gesamtgewicht des Tränkungsmittels vor.

Die erfindungsgemäßen Tränkungsmittel sind frei von Parabenen. Ebenso ist das Reinigungstuch, das kosmetische Produkt, bestehend aus Tränkungsmittel und Tuch und auch die Verpackungseinheit für das Reinigungstuch frei von Parabenen.

Parabene im Sinne der vorliegenden Erfindung haben folgende allgemeine Strukturformel:

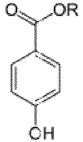

R bedeutet eine Alkylseitenkette, die aus einer unverzweigten oder verzweigten Kette von 1 bis 5 Kohlenstoffatomen oder aus aromatischen Resten bestehen kann. Bekannte Parabene sind Methyl-, Ethyl-, Propyl- Butylparaben, ebenso wie Isopropyl-, Isobutyl-, Pentyl- und Phenylparaben. Benzylparaben ist in kosmetischen Mitteln als Konservierungsmittel nicht zugelassen. Die erfindungsgemäßen Zubereitungen sind frei von Parabenen. Im Sinne der vorliegenden Erfindung bedeutet frei von Parabenen, dass den Zubereitungen keine Parabene zugesetzt werden und auch keine Parabene über konservierte Inhaltsstoffe eingeschleppt werden. Dies ist gleichbedeutend mit der Aussage, dass die erfindungsgemäßen Zubereitungen keine Parabene enthalten.

Im Sinne der vorliegenden Erfindung bedeutet frei von, dass weniger als 0,001 Gew.-%, bevorzugt weniger als 0,0001 Gew.-%, besonders bevorzugt 0 Gew.-% der jeweiligen Substanz(en), bezogen auf das Gesamtgewicht der Zubereitung, vorliegen.

In einer vorteilhaften Ausführungsform sind im erfindungsgemäßen Tränkungsmittel und auch im gesamten erfindungsgemäßen Reinigungsmittel keine "klassischen Tenside" enthaltenen, d.h. es werden keine klassischen Tenside zugesetzt und die klassischen Tenside werden auch nicht durch andere Rohstoffe, die diese enthalten, eingeschleppt. Unter klassischen Tensiden werden oberflächenaktive Substanzen verstanden, die gewöhnlich in Reinigungszubereitungen, auch in Tränkungsmittel von Reinigungstüchern, eingearbeitet werden. Derartige Tenside werden oftmals in anionische, amphotere, nichtionische und kationische Tenside eingeteilt. Beispielhaft seien im Folgenden häufig verwendete Vertreter genannt: Anionische Tenside:
Acylaminosäuren (und deren Salze), wie beispielsweise
- Acylglutamate, beispielsweise Natriumacylglutamat, Natrium Cocoylglutamat, Di-TEApalmitoylaspartat und Natrium Caprylic/ Capric Glutamat,
- Sarcosinate, beispielsweise Myristoyl Sarcosin, TEA-lauroyl Sarcosinat, Natriumlauroylsarcosinat und Natriumcocoylsarkosinat,
- Alaninate,
- Glycinate.

Carbonsäuren und Derivate, wie beispielsweise
- Carbonsäuren, beispielsweise Laurinsäure, Aluminiumstearat, Magnesiumalkanolat und Zinkundecylenat, Stearinsäure/-salz, Palmitinsäure/-salz,
- Ester-Carbonsäuren, beispielsweise Calciumstearoyllactylat, Laureth-6-Citrat und Natrium PEG-4-Lauramidcarboxylat,
- Ether-Carbonsäuren, beispielsweise Natriumlaureth-13-Carboxylat und Natrium PEG-6-Cocamide Carboxylat,
- Acyllactylate, Lauroyllactylat, Caproyllactylat.

### Phosphorsäureester und Salz.

Sulfonsäuren und Salze, wie beispielsweise
- Acyl-isethionate, z.B. Natrium-/ Ammoniumcocoyl-isethionat, Natrium Lauryl Methylisethionate,
- Alkylarylsulfonate,
- Alkylsulfonate, beispielsweise Natriumcocosmonoglyceridsulfat, Natrium C12-14 Olefin-sulfonat, Natriumlaurylsulfoacetat und Magnesium PEG-3 Cocamidsulfat,
- Sulfosuccinate, beispielsweise Dioctylnatriumsulfosuccinat, Dinatriumlaurethsulfosuccinat, Dinatriumlaurylsulfosuccinat, Dinatriumundecylenamido-MEA-Sulfosuccinat und PEG-5 Laurylcitrat Sulfosuccinat,
- Taurate, beispielsweise Natriumlauroyltaurat und Natriummethylcocoyltaurat. Schwefelsäureester, wie beispielsweise
- Alkylethersulfat mit unterschiedlichen Ethoxylierungsgraden und deren Gemische,
- Alkylsulfate.

Kationische Tenside sind beispielsweise
- Alkylamine,
- Alkylimidazole,
- Ethoxylierte Amine
- Quaternäre Tenside, beispielsweise Cetyl Trimethylammoniumhalogenid.
- Esterquats, beispielsweise Dicocoylethyl Hydroxyethylmoium Methosulfate und
- Amidquats, beispielsweise Palmitamidopropyltrimonium Chlorid.

Amphotere Tenside sind beispielsweise
- Acyl-/dialkylethylendiamin, beispielsweise Natriumacylamphoacetat, Dinatriumacylamphodipropionat, Dinatriumalkylamphodiacetat, Dinatrium Cocoamphodiacetat, Dinatrium Cocoamphomonoacetat, Natriumacylamphohydroxypropylsulfonat, Dinatriumacylamphodiacetat und Natriumacylamphopropionat,
- N-Alkylaminosäuren, beispielsweise Aminopropylalkylglutamid, Alkylaminopropionsäure, Natriumalkylimidodipropionat und Lauroamphocarboxyglycinat.
- Betaine, beispielsweise Coco Betaine, Cocoamidopropyl Betaine,
- Sultaine, beispielsweise Lauryl Hydroxy Sultaine.

Nichtionische Tenside sind beispielsweise
- Alkanolamide, wie Cocamide MEA/ DEA/ MIPA,
- Aminoxide, wie Cocoamidopropylaminoxid,
- Ether, beispielsweise ethoxylierte/propoxylierte Alkohole, Laureth-X mit X = 2 bis 10, wobei X Ethoxygruppe bedeutet, ethoxylierte/ propoxylierte Ester, ethoxylierte/ propoxylierte Glycerinester, PEG-200 Hydrogenated Glyceryl Palmat, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.
- Sucroseester, -Ether
- Polyglycerinester, Diglycerinester, Monoglycerinester
- Methylglucosester, Ester von Hydroxysäuren.

Die erfindungsgemäßen Tränkungsmittel sind frei von den genannten Tensiden.

Hydrodispersionen stellen Dispersionen einer flüssigen, halbfesten oder festen inneren (diskontinuierlichen) Lipidphase in einer äußeren wässrigen (kontinuierlichen) Phase dar. Hydrodispersionen sind - wie auch Emulsionen, die sich durch eine ähnliche Phasenanordnung auszeichnen - metastabile Systeme und daher geneigt, in einen Zustand zweier in sich zusammenhängender diskreter Phasen überzugehen. In einer klassischen O/W-Emulsion verhindert die Wahl eines geeigneten Emulgators die Phasentrennung. Im Gegensatz zu den klassischen Emulsionen enthalten Hydrodispersionen aber nur sehr geringe Mengen an Emulgatoren (bis zu 0,1 Gew.-%, bevorzugt bis 0,05 Gew.-%) bzw. können sogar gänzlich frei von Emulgatoren sein.

Bei Hydrodispersionen, welche aus einer flüssigen Lipidphase in einer äußeren wässrigen Phase bestehen, wird die Stabilität des Mehrphasensystems üblicherweise dadurch gewährleistet, dass in der wässrigen Phase mit Hilfe eines Gelbildners bzw. Verdickungsmittels ein Gelgerüst aufgebaut wird, in welchem die Lipidtröpfchen stabil suspendiert sind. Der Gelbildner bzw. das Verdickungsmittel kann eine polymere Verbindung sein, die stabilisierend wirkt. Geeignete stabilisierend wirkende Polymere können Homopolymere oder Copolymere von Acrylsäure und/oder Acrylamid und deren Derivaten sein.

Das wenigstens eine stabilisierend wirkende Polymer kann vorteilhaft ausgewählt aus der Gruppe der Acrylates/C10-30 Alkyl Acrylate Crosspolymere. Diese sind erhältlich unter den Handelsbezeichnungen Carbopol^{®} Ultrez 20, Carbopol^{®} Ultrez 21, Pemulen TR1, Pemulen TR2, Carbopol^{®} 1342, Carbopol^{®} 1382, Carbopol^{®} ETD 2020 von der Firma Lubrizol.

In dem erfindungsgemäßen Tränkungsmittel ist die wenigstens eine polymere Verbindung, die stabilisierend wirkt, mit einem Gesamtgehalt von 0,02 bis 0,15 Gew.-%, bevorzugt 0,03 bis 0,1 Gew.-% enthalten, bezogen auf das Gesamtgewicht des Tränkungsmittels.

Im Sinne der vorliegenden Erfindung sind polymere Verbindungen, die filmbildend wirken , im Folgenden als Filmbildner bezeichnet, Stoffe mit unterschiedlicher Zusammensetzung, die durch die folgende Eigenschaft charakterisiert sind: Löst man einen Filmbildner in Wasser oder anderen geeigneten Lösungsmitteln, so bildet sich nach dem Verdunsten des Lösemittels einen Film aus. Geschieht das Verdunsten des Lösungsmittels nach Auftrag einer Filmbildner enthaltenden Lösung/Zusammensetzung auf der Haut oder den Haaren, so bildet sich auf der Oberfläche der Haut oder der Haare ein Film aus, der im Wesentlichen eine Schutzfunktion hat.

Ein weiterer Typ von Filmbildnern umfasst Substanzen, die in einem Lösemittel, welches nicht notwendigerweise verdunsten muss, nach dem Auftragen Filme bilden. In einem solchen Falle handelt es sich zumeist um öllösliche polymere Grundkörper.

Es ist von Vorteil, die Filmbildner aus der Gruppe der Polymere auszuwählen, die als Monomere Vinylpyrrolidone enthalten, das durch die folgende Formel dargestellt werden kann:

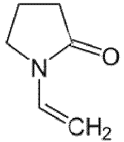

Dies können Homopolymere wie das Polyvinylpyrrolidone (PVP) oder Copolymere sein. Von den Copolymere insbesondere vorteilhaft sind beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind.

In dem erfindungsgemäßen Tränkungsmittel ist die wenigstens eine polymere Verbindung, die filmbildend wirkt, mit einem Gesamtgehalt von 0,2 bis 1,0 Gew.-%, bevorzugt 0,3 bis 0,6 Gew.-% enthalten, bezogen auf das Gesamtgewicht des Tränkungsmittels.

Es sind Polymere bekannt, die filmbildend und zugleich stabilisierend wirken. Zuweilen ist die stabilisierende Eigenschaft stärker ausgeprägt als die filmbildende und umgekehrt. Auch gibt es Vorbeschreibungen, die den Polymeren, die in dieser Anmeldung als stabilisierend eingeordnet werden, filmbildende Eigenschaften zuschreiben oder Polymere, die in dieser Anmeldung als filmbildend klassifiziert werden, werden auch stabilisierende Eigenschaften zugeschrieben. Es ist jedoch für die vorliegende Erfindung wesentlich, dass in dem Tränkungsmittel eine Substanz/Substanzen mit stabilisierenden Eigenschaften (für die Ausbildung der Hydrodispersion) und filmbildenden Eigenschaften (zur Ausbildung eines Schutzfilmes) enthalten sind.

In dem erfindungsgemäßen Tränkungsmittel sind vorteilhaft ein Acrylates/C10-30 Alkyl Acrylate Crosspolymer und ein PVP Hexadecen Copolymer enthalten; weiter vorteilhaft sind in Bezug auf die wenigstens zwei polymeren Verbindungen, wobei wenigstens eine polymere Verbindung stabilisierend und wenigstens eine polymere Verbindung filmbildend wirkt oder wobei wenigstens eine polymere Verbindung stabilisierend und filmbildend wirkt und eine weitere polymere Verbindung enthalten ist, nur ein Acrylates/C10-30 Alkyl Acrylate Crosspolymer und ein PVP Hexadecen Copolymer enthalten.

In dem erfindungsgemäßen Tränkungsmittel ist somit der Gesamtgehalt an den wenigstens zwei polymere Verbindungen, wobei wenigstens eine polymere Verbindung stabilisierend und wenigstens eine polymere Verbindung filmbildend wirkt oder wobei wenigstens eine polymere Verbindung stabilisierend und filmbildend wirkt und eine weitere polymere Verbindung enthalten ist, aus dem Bereich von 0,22 bis 1,2 Gew.-%, bevorzugt 0,33 bis 0,7 Gew.-% zu wählen, bezogen auf das Gesamtgewicht des Tränkungsmittels.

Das erfindungsmäße Tränkungsmittel kann zusätzlich zu den Substanzen, die gleichzeitig pflegend wirken und die Konservierung unterstützen, wenigstens ein weiteres Feuchthaltemittel enthalten. Als weiteres Feuchthaltemittel werden Stoffe oder Stoffgemische verstanden, welche kosmetischen Zubereitungen die Eigenschaft verleihen, nach dem Auftragen bzw. Verteilen auf der Haut den Feuchtigkeitsgehalt der Haut, insbesondere der Epidermis positiv zu beeinflussen und/oder zu regulieren. Vorteilhafte weitere Feuchthaltemittel im Sinne der vorliegenden Erfindung sind beispielsweise Glycerin, Milchsäure, Harnstoff und/oder Sorbitol, insbesondere Glycerin.

Wenn in dem erfindungsgemäßen Tränkungsmittel wenigstens ein weiteres Feuchthaltemittel enthalten ist, so liegt der Gesamtgehalt des wenigstens einen Feuchthaltemittels bei 0,1 bis 10 Gew.-%, bevorzugt 0,2 bis 2,0 Gew.-%, bezogen auf das Gesamtgewicht des Tränkungsmittels vor.

Das erfindungsmäße Tränkungsmittel kann vorteilhaft wenigstens ein Emollient enthalten. Unter einem Emollient werden Substanzen verstanden, die die Haut weich und geschmeidig machen. Es handelt sich dabei vorteilhaft um eine lipophile Komponente. Insbesondere vorteilhaft kann die lipophile Komponente aus der Gruppe der Monoester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen ausgewählt werden. Solche Esteröle können dann weiter insbesondere vorteilhaft gewählt werden aus der Gruppe 2-Ethylhexyl Benzoate, 2 Ethylhexyl Hydroxystearate Benzoate, 2-Ethylhexyl Laurate, 2-Ethylhexyl Palmitate, 2-Ethylhexyl Stearate, 2-Hexyldecyl Stearate, 2-Octyldodecyl Palmitate, Butyloctyl Salicylate, C12-15 Alkyl Benzoate, Castor Oil Benzoate, Cetearyl Isononanoate, Coco Caprylate/Caprate, Decyl Oleate, Diethylhexyl Adipate, Dipropylene Glycol Dibenzoate, Ethylhexyl Cocoate, Glyceryl Caprate, Glyceryl Isostearate, Isononyl Isononanoate, Isononyl Stearate, Isooctyl Stearate, Isopropyl Isostearate, Isopropyl myristate, Isopropyl Oleate, Isopropyl Palmitate, Isopropyl Stearate, Isostearyl Palmitate, Myristyl Myristate, n-Butyl Stearate, n-Decyl Oleate, n-Hexyllaurat, Octyl Cocoate, Octyl Isostearate, Octyl Palmitate, Octyldodeceyl Myristate, Oleyl Oleate, Propylheptyl Caprylate, Stearyl Heptanoate, Tridecyl Stearate und/oder Triisostearin.

Ferner kann die lipophile Komponente vorteilhaft gewählt werden aus der Gruppe der Dialkylether und Dialkylcarbonate, vorteilhaft sind z. B. Dicaprylylether (beispielsweise erhältlich unter der Handelsbezeichnung Cetiol OE) und/oder Dicaprylylcarbonat (beispielsweise erhältlich unter der Handelsbezeichnung Cetiol CC).

Auch beliebige Abmischungen der genannten lipophilen Komponenten sind vorteilhaft im Sinne der vorliegenden Erfindung einzusetzen.

Besonders bevorzugt sind die Verbindungen Isopropyl Stearate, Isopropyl Palmitate und/oder Dicaprylylether.

In dem erfindungsgemäßen Tränkungsmittel liegt das wenigstens eine Emollient vorteilhaft mit einem Gesamtgehalt von 0,05 bis 5,0 Gew. %, bevorzugt 0,1 bis 3,5 Gew. %, bezogen auf das Gesamtgewicht des Tränkungsmittels, vor.

Es ist weiterhin auch ebenso vorteilhaft, wenn das erfindungsmäße Tränkungsmittel zusätzlich wenigstens einen kosmetischen Wirkstoff ausgewählt aus der Gruppe der Vitamine, Pflanzenextrakte und/oder Einzelwirkstoffe enthält.

Das Tränkungsmittel kann erfindungsgemäß zusätzlich kosmetische Hilfsstoffe enthalten, wie sie üblicherweise in solchen Mitteln verwendet werden, z.B. Komplexbildner und Farbstoffe, Antioxidantien sowie Substanzen, um den pH-Wert zu einzustellen.

Die erfindungsgemäßen Tränkungsmittel sind frei von Silikonen. Ebenso ist das Reinigungstuch, das kosmetische Produkt, bestehend aus Tränkungsmittel und Tuch und auch die Verpackungseinheit für das Reinigungstuch frei von Silikonen.

Die Begriffe Silikon oder Silikonverbindung werden synonym verwendet. Silikonverbindungen zeichnen sich durch das Vorhandensein von Silicium- und Sauerstoffatomen aus, die miteinander verknüpft sind und eine Vielzahl verschiedener Verbindungen ausbilden können.

Im Sinne der vorliegenden Erfindung bedeutet der Begriff "frei von", dass weniger als 0,001 Gew.-%, bevorzugt weniger als 0,0001 Gew.-%, besonders bevorzugt 0 Gew.-% der jeweiligen Substanz(en), bezogen auf das Gesamtgewicht des Tränkungsmittels, vorliegen.

Das Tuchmaterial, welches für die Reinigungstücher verwendet wird, ist dadurch gekennzeichnet, dass es aus Fasern besteht, die natürlichen Ursprungs und biologisch abbaubar sind. Die genannten Tücher können glatt oder auch oberflächenstrukturiert sein. Erfindungsgemäß bevorzugt sind oberflächenstrukturierte Tücher.

**Erfindungsgemäß** bevorzugt werden in Kombination mit den erfindungsgemäßen Tränkungsmitteln "trockene" Tücher eingesetzt, welche aus Vlies bestehen, insbesondere aus wasserstrahlverfestigten und/oder wasserstrahlgeprägten Vlies.

Derartige Vliese können Makroprägungen jeden gewünschten Musters aufweisen. Die zu treffende Auswahl richtet sich zum einen nach der aufzubringenden Tränkung und zum anderen nach dem Einsatzfeld, auf dem das spätere Tuch Verwendung finden soll.

Werden geprägte Vliese verwendet, so erleichtern große Kavitäten an der Vliesoberfläche und im Vlies die Aufnahme von Schmutz und Verunreinigungen, wenn mit dem getränkten Tuch über die Haut gefahren wird. Die Reinigungswirkung kann gegenüber ungeprägten Tüchern um ein Vielfaches gesteigert werden.

Es hat sich für das Tuch als vorteilhaft herausgestellt, wenn dieses ein Gewicht von 30 bis 120 g/m², vorzugsweise von 35 bis 80 g/m², besonders bevorzugt 40 bis 65 g/m² hat (gemessen bei 20 C ± 2°C und bei einer Feuchtigkeit der Raumluft von 65 % ± 5 % für 24 Stunden).

Als Ausgangsmaterialien für den Vliesstoff des Tuches wird ein Faserstoff auf natürlicher Basis gewählt. Beispielhaft seien Viskose, Baumwolle, Zellulose, Jute, Hanf, Sisal, Seide, Wolle, angeführt.

In einer weiteren besonders vorteilhaften Ausführungsform bestehen die Fasern zu 100% aus Viskose mit unterschiedlichen Feinheiten. Die Feinheiten der Fasern betragen 1,0 dTex bis 2,0 dTex bevorzugt 1,2 dTex bis 1,8dTex. Derartige Tücher sind beispielsweise erhältlich von der Firma Jacob Holm unter der Bezeichnung10.045.016 oder von der Firma Spuntech unter der Bezeichnung 13P46V100.

Vorteilhaft sind ebenfalls Fasern aus Viskose, Lyocel, Polylactid acid und Baumwolle in unterschiedlichen Zusammensetzungen, die u.a. von der Firma Lenzing unter der Bezeichnung LENZING^{™} Lyocell und LENZING^{™} Viscose oder von der Firma Suominen unter der Bezeichnung Biolace und Fibrella angeboten werden.

Die erfindungsgemäß vorteilhaft einsetzbaren Tücher sind hinsichtlich ihrer biologischen Abbaubarkeit untersucht worden. Es konnte eine biologische Abbaubarkeit nach OECD Richtlinie 301 B gezeigt werden.

Für das erfindungsgemäße Reinigungsprodukt, also das Tuch (biodegradable, non-woven, am Beispiel von Tuch Jacob Holm unter der Bezeichnung 10.040.007) getränkt mit dem Tränkungsmittel gemäß Beispiel 3 wurde die biologische Abbaubarkeit gemäß OECD Richtlinie 301 B nachgewiesen.

Um die biologische Abbaubarkeit einer Zusammensetzung, beispielsweise des Tränkungsmittels, zu bestimmen, wird auf Daten der OECD oder auf gleichwertige Methoden zur Klassifizierung der Inhaltsstoffe zurückgegriffen. Dabei wird der Prozentsatz der biologisch abbaubaren Inhaltstoffe einer Zusammensetzung berechnet, indem das Gewicht der biologisch abbaubaren organischen Inhaltsstoffe durch das Gesamtgewicht der organischen Inhaltsstoffe dividiert wird. Es ergeben sich Werte wie beispielsweise zu XX% biologisch abbaubare Zusammensetzung.

Für das Tränkungsmittel gemäß Beispielrezeptur 3 wurde eine biologische Abbaubarkeit von 98 % errechnet.

Der Begriff der biologischen Abbaubarkeit beschreibt den Prozess des Abbaus von organischen Verbindungen durch Lebewesen, insbesondere Saprobionten. Im Idealfall entstehen anorganische Stoffe, wie beispielsweise CO₂, O₂ und Ammoniak; Verbindungen, die von Pflanzen und Mikroorganismen wieder für den Aufbau von organischen Verbindungen genutzt werden.

Organische, chemische Verbindungen, die leicht biologisch abbaubar sind, sind nach OECD 301, für kosmetische Zusammensetzungen meist nach OECD 301 B klassifiziert. Diese Substanzen oder Zusammensetzungen können rasch und vollständig abgebaut werden.

Organische, chemische Verbindungen, die nach OECD 302 klassifiziert sind, sind zwar eingeschränkt, jedoch grundsätzlich biologisch abbaubar.

Darüber hinaus können die Fasern auch eingefärbt sein, um die optische Attraktivität des Vlieses betonen und/oder erhöhen zu können. Die Fasern können zusätzlich UV-Stabilisatoren und/oder Konservierungsmittel enthalten.

Vorteilhafte Tücher im Sinne der vorliegenden Erfindung haben eine Reißkraft von insbesondere

| | | [N/50mm] |
|---|---|---|
| im trockenen Zustand | Maschinenrichtung | >40, vorzugsweise >60 |
| | Querrichtung | >4, vorzugsweise >9 |

Die Dehnfähigkeit eines vorteilhaften Tuches beträgt vorzugsweise

| | | |
|---|---|---|
| im trockenen Zustand | Maschinenrichtung | 2 % bis 30 %, bevorzugt 5 % bis 30 % |
| | Querrichtung | 50 % bis 180 %, bevorzugt 60 % bis 175 % |

Das Gewichtsverhältnis des ungetränktem Tuch zu dem Tränkungsmittel beträgt erfindungsgemäß vorteilhaft 1:1 bis 1:5, besonders vorteilhaft 1:1,5 bis 1:4,5, insbesondere vorteilhaft 1: 1,75 bis 1:3,5.

Das Reinigungsprodukt, bestehend aus dem erfindungsgemäßen Tuch und dem erfindungsgemäßen Tränkungsmittel wird vorteilhaft in einer Verpackung angeboten, die zu 30 %, bevorzugt 45 % aus recyceltem Plastikmaterial besteht. Weiter vorteilhaft besteht das Verpackungsmaterial aus Polyester- und Polyethylen-Verbund.

Die Reinigungsleistung und auch die Milde eines erfindungsgemäßen Reinigungsmittels wurden in einem PIU (Product In Use-Test) abgefragt. Sowohl in Bezug auf die Reinigungsleistung als auch auf die Milde des erfindungsgemäßen Reinigungsmittels wurde bestätigt, dass sie auf dem gleichen Niveau liegen, wie die Reinigungsleistung und Milde eines Reinigungstuchs bestehend aus einem Paraben-haltigen Tränkungsmedium und einem nicht bioabbaubaren Vlies.

Für die Studie verwendeten 160 Frauen, im Alter von 20 bis 60 Jahren mindestens einmal pro Tag ein Gesichtsreinigungstuch über einen Zeitraum von 2 Wochen. Die Testteilnehmerinnen waren Verwenderinnen von wasserfesten Make-up-Produkten, auch von wasserfester Mascara.

Die Bewertung erfolgte auf eine Fünfer-Skala: 0 Punkte bedeutet "stimmt überhaupt nicht", 5 Punkte "stimmt vollständig".

Die Antworten als vier und fünf Punkte wurden zusammengezählt. Dies ergab folgende Ergebniszustimmung in Prozent umgerechnet.

| | **N2** | **N7** |
|---|---|---|
| | Tränkungsmittel mit Parabenen; Tuch: non-woven, nicht biologisch abbaubar | Tränkungsmittel ohne Parabene; Tuch: non-woven, biologisch abbaubar |
| Reinigt dein Gesicht besonders gründlich | 87 | 87 |
| Entfernt wasserfestes Make-up gründlich | 87 | 94 |
| Pflegt meine Haut besonders intensiv | 76 | 77 |
| Ist für empfindliche Haut geeignet | 73 | 70 |

Die Tränkungsmittel N2 und N7 haben folgende Zusammensetzung, die Angaben sind in Gew.-% und beziehen sich auf Aktivgehalte, wenn nicht anders angegeben.

| INCI Bezeichnung | **N2** | **N7** |
|---|---|---|
| Aqua | Ad 100% | Ad 100% |
| Isopropyl Stearate | 2,5 | 2,5 |
| Glycerin | 1 | 1 |
| Phenoxyethanol | 0,8 | 0,8 |
| VP/Hexadecene Copolymer | 0,3 | 0,3 |
| Parfum | 0,4 | 0,4 |
| Methylparaben | 0,3 | 0 |
| Ethylhexylglycerin | 0,2 | 0,15 |
| Acrylates/C10-30 Alkyl Acrylate Cross-polymer | 0,06 | 0,06 |
| Sodium Hydroxide | q.s.* | q.s.* |
| Decylene Glycol | 0 | 0,15 |
| Nelumbo Nucifera Flower Extract | 0 | 0,01 |

| | | |
|---|---|---|
| * zur Einstellung des pH-Wertes, pH-Wertbereich: 6,0-6,5. Beim Reinigungsmittel N2 ist das Tuch ist eine Mischung aus 60% Polyester und 40% Viskose; Beim Reinigungsmittel N7 Tuch besteht aus 100% Viskose. | | |

N7 stellt das erfindungsgemäße Reinigungsprodukt dar. Die Ergebnisse zeigen, dass das erfindungsgemäße Reinigungsprodukt in Bezug auf die gründliche Reinigung und Pflege genauso gut bewertet wird, wie ein herkömmliches Produkt. Die Eignung für empfindliche Haut wird etwas weniger gut bewertet im Vergleich zum herkömmlichen Produkt. In Bezug auf die Entfernung von wasserfestem Make-up wird das erfindungsgemäße Produkt besser bewertet als das das herkömmliche Produkt. Insgesamt ist das erfindungsgemäße Produkt in der Reinigungsleistung genauso gut bis besser als das herkömmliche Produkt.

Um die Verträglichkeit zu beurteilen, wurde parallel ein Verträglichkeitstest unter Beobachtung eines Hausarztes und Augenarztes durchgeführt. Die Teilnehmerinnen, die am oben beschriebenen Product-in-Use-Test teilnahmen, nahmen auch am Verträglichkeitstest teil. Für das erfindungsgemäße Reinigungsmittel (N7) wurde eine gute Hautakzeptanz für Gesicht, Augenlider und Lippen sowie eine gute Augenverträglichkeit attestiert.

### Beispiele:

Die Beispiele sollen die vorliegende Erfindung erläutern.

Die Zahlenangaben beziehen sich auf Gewichtsprozent und den jeweiligen Aktivgehalt, soweit nicht anders angegeben.

| INCI Bezeichnung | **1** | **2** | **3** | **4** |
|---|---|---|---|---|
| Wasser | ad 100% | ad 100% | ad 100% | ad 100% |
| Isopropyl Stearate | | 3 | 3 | |
| Isopropyl Palmitate | 2 | | | 2 |
| Dicrapylyl Ether | 1 | | | |
| Glycerin | 0,87 | 0,87 | 1 | 1,5 |
| Phenoxyethanol | 0,8 | 0,8 | 0,9 | 0,9 |
| VP/Hexadecene Copolymer | 0,5 | 0,4 | 0,5 | 0,5 |
| Hydroxyacetophenone | 0,23 | 0,23 | 0 | 0,17 |
| Ethylhexylglycerin | 0,23 | 0,15 | 0,23 | 0,15 |
| Acrylates/C10-30 Alkyl Acrylate Crosspolymer | 0,1 | 0,1 | 0,05 | 0,05 |
| Tocopheryl Acetate | 0,06 | | | 0,06 |
| Sodium Hydroxide | q.s.* | q.s.* | q.s.* | q.s.* |
| Panthenol | 0,013 | | | 0,1 |
| Decylene Glycol | | | 0,1 | 0,1 |
| Nelumbo Nucifera Flower Extract | 0,01 | 0,01 | 0,01 | 0 |
| Parfum | 0 | 0,3 | 0,3 | 0 |

| | | | | |
|---|---|---|---|---|
| * zur Einstellung des pH-Wertes, pH-Wertbereich 6,0 bis 6,5. | | | | |

## Patentansprüche

1. Reinigungsmittel bestehend aus einem Tuch, **dadurch gekennzeichnet, dass** das Tuch aus Fasern besteht, die natürlichen Ursprungs und biologisch abbaubar sind und einem Tränkungsmittel, dadurch charakterisiert, dass das Tränkungsmittel in Form einer Hydrodispersion vorliegt, die Silikon-frei und Paraben-frei ist, das heißt weniger als 0,001 Gewichts-%, bezogen auf das Gesamtgewicht des Tränkungsmediums enthält, enthaltend
- Phenoxyethanol,
- wenigstens zwei Substanzen, die gleichzeitig pflegend wirken und die Konservierung unterstützen und
- wenigstens zwei polymere Verbindungen, wobei wenigstens eine polymere Verbindung stabilisierend und wenigstens eine polymere Verbindung filmbildend wirkt oder wobei wenigstens eine polymere Verbindung stabilisierend und filmbildend wirkt und wenigstens eine weitere polymere Verbindung enthalten ist,
- **dadurch gekennzeichnet, dass** die wenigstens zwei Substanzen, die gleichzeitig pflegend wirken und die Konservierung unterstützen, ausgewählt werden aus der Gruppe Ethylhexylglycerin, Hydroxyacetophenone und Decylene Glycol.

2. Reinigungsmittel nach Anspruch 1, **dadurch gekennzeichnet, dass** das Tränkungsmittel Wasser mit einem Gehalt von 80 bis 99 Gew.-%, bevorzugt 93 bis 98,9 Gew.-%, bezogen auf das Gesamtgewicht des Tränkungsmittels, enthält.

3. Reinigungsmittel nach Anspruch 1 und/oder 2, **dadurch gekennzeichnet, dass** in dem Tränkungsmittel Phenoxyethanol mit einem Gehalt von 0,5 bis 0,98 Gew.-%, bevorzugt 0,8 bis 0,95 Gew.-% enthalten ist, bezogen auf das Gesamtgewicht des Tränkungsmittels.

4. Reinigungsmittel nach wenigstens einem der vorstehenden Ansprüche, **dadurch gekennzeichnet, dass** in dem Tränkungsmittel als Substanzen, die gleichzeitig pflegend wirken und die Konservierung unterstützen, die Kombination von Ethylhexylglycerin und Decylene Glycol einigesetzt wird.

5. Reinigungsmittel nach wenigstens einem der vorstehenden Ansprüche, **dadurch gekennzeichnet, dass** in dem Tränkungsmittel die wenigstens zwei Substanzen, die gleichzeitig pflegend wirken und die Konservierung unterstützen, mit einem Gesamtgehalt von 0,15 bis 0,6 Gew.-%, bevorzugt 0,3 bis 0,45 Gew.-% enthalten sind, bezogen auf das Gesamtgewicht des Tränkungsmittels.

6. Reinigungsmittel nach wenigstens einem der vorstehenden Ansprüche, **dadurch gekennzeichnet, dass** das Tränkungsmittel frei ist von anionischen, amphoteren, nichtionischen und/oder kationischen Tensiden.

7. Reinigungsmittel nach wenigstens einem der vorstehenden Ansprüche, **dadurch gekennzeichnet, dass** für das Tränkungsmittel die wenigstens eine polymere Verbindung, die stabilisierend wirkt, ausgewählt wird aus der Gruppe Homopolymere oder Copolymere von Acrylsäure und/oder Acrylamid und deren Derivaten, bevorzugt aus der Gruppe der Acrylates/C10-30 Alkyl Acrylate Crosspolymere.

8. Reinigungsmittel nach wenigstens einem der vorstehenden Ansprüche, **dadurch gekennzeichnet, dass** in dem Tränkungsmittel die wenigstens eine polymere Verbindung, die stabilisierend wirkt mit einem Gesamtgehalt von 0,02 bis 0,15 Gew.-%, bevorzugt 0,03 bis 0,1 Gew.-% enthalten ist, bezogen auf das Gesamtgewicht des Tränkungsmittels.

9. Reinigungsmittel nach wenigstens einem der vorstehenden Ansprüche, **dadurch gekennzeichnet, dass** für das Tränkungsmittel die wenigstens eine polymere Verbindung, die filmbildend wirkt, ausgewählt wird aus der Gruppe der Polymere enthaltend Vinylpyrrolidone-Monomere, bevorzugt aus der Gruppe der Copolymere des Polyvinylpyrrolidons, weiter bevorzugt aus PVP Hexadecen Copolymer und/oder PVP Eicosen Copolymer.

10. Reinigungsmittel nach wenigstens einem der vorstehenden Ansprüche, **dadurch gekennzeichnet, dass** in dem Tränkungsmittel die wenigstens eine polymere Verbindung, die filmbildend wirkt mit einem Gesamtgehalt von 0,2 bis 1,0 Gew.-%, bevorzugt 0,3 bis 0,6 Gew.-% enthalten ist, bezogen auf das Gesamtgewicht des Tränkungsmittels.

11. Reinigungsmittel nach wenigstens einem der vorstehenden Ansprüche, **dadurch gekennzeichnet, dass** in dem Tränkungsmittel die wenigstens zwei polymere Verbindungen, wobei wenigstens eine polymere Verbindung stabilisierend und wenigstens eine polymere Verbindung filmbildend wirkt oder wobei wenigstens eine polymere Verbindung stabilisierend und filmbildend wirkt und wenigstens eine weitere polymere Verbindung enthalten ist, mit einem Gesamtgehalt von 0,22 bis 1,2 Gew.-%, bevorzugt 0,33 bis 0,7 Gew.-% enthalten sind, bezogen auf das Gesamtgewicht des Tränkungsmittels.

12. Reinigungsmittel nach wenigstens einem der vorstehenden Ansprüche, **dadurch gekennzeichnet, dass** in dem Tränkungsmittel wenigstens ein Emollient enthalten ist, bevorzugt ausgewählt aus Monoestern aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, Estern aus aromatischen Carbonsäuren und gesättigten und/oder ungesättigten, verzweigten und/oder unverzweigten Alkoholen einer Kettenlänge von 3 bis 30 C-Atomen, Dialkylethern und/oder Dialkylcarbonaten.

13. Reinigungsmittel nach wenigstens einem der vorstehenden Ansprüche, **dadurch gekennzeichnet, dass** in dem Tränkungsmittel das wenigstens ein Emollient mit einem Gesamtgehalt von 0,05 bis 5,0 Gew. %, bevorzugt 0,1 bis 3,5 Gew. % enthalten ist, bezogen auf das Gesamtgewicht des Tränkungsmittels.

14. Reinigungsmittel nach wenigstens einem der vorstehenden Ansprüche, **dadurch gekennzeichnet, dass** das Tränkungsmittel frei von Silikonen ist.

15. Reinigungsmittel nach wenigstens einem der vorstehenden Ansprüche, **dadurch gekennzeichnet, dass** das Tuch aus einem Faserstoff auf natürlicher Basis besteht, bevorzugt aus Viskose, Baumwolle, Zellulose, Jute, Hanf, Sisal, Seide, Wolle oder Mischungen davon.

16. Reinigungsmittel nach wenigstens einem der vorstehenden Ansprüche, **dadurch gekennzeichnet, dass** das Tuch zu 100% aus Viskose besteht, bevorzugt mit den Feinheiten 1,0 dTex bis 2,0 dTex, weiter bevorzugt 1,2 dTex bis 1,8dTex.

17. Reinigungsmittel nach wenigstens einem der vorstehenden Ansprüche, **dadurch gekennzeichnet, dass** das Gewichtsverhältnis des ungetränktem Tuchs zu dem Tränkungsmittel 1:1 bis 1:5, besonders vorteilhaft 1:1,5 bis 1:4,5, insbesondere vorteilhaft 2,0:3,5 beträgt.

## Claims

1. Cleansing product consisting of a wipe, **characterized in that** the wipe consists of fibres which are of natural origin and biodegradable and an impregnation agent, **characterized in that** the impregnation agent is in the form of a hydrodispersion which is silicone-free and paraben-free, that is to say contains less than 0.001% by weight, based on the total weight of the impregnation medium, containing
- phenoxyethanol,
- at least two substances which simultaneously have a caring effect and support preservation and
- at least two polymeric compounds, where at least one polymeric compound has a stabilizing effect and at least one polymeric compound has a film-forming effect or where at least one polymeric compound has a stabilizing and film-forming effect and at least one further polymeric compound is present,
- **characterized in that** the at least two substances which simultaneously have a caring effect and support preservation are selected from the group of Ethylhexylglycerin, Hydroxyacetophenone and Decylene Glycol.

2. Cleansing product according to Claim 1, **characterized in that** the impregnation agent contains water in a content of 80% to 99% by weight, preferably 93% to 98.9% by weight, based on the total weight of the impregnation agent.

3. Cleansing product according to Claim 1 and/or 2, **characterized in that** phenoxyethanol is present in the impregnation agent in a content of 0.5% to 0.98% by weight, preferably 0.8% to 0.95% by weight, based on the total weight of the impregnation agent.

4. Cleansing product according to at least one of the preceding claims, **characterized in that** in the impregnation agent the combination of Ethylhexylglycerin and Decylene Glycol is used as substances which simultaneously have a caring effect and support preservation.

5. Cleansing product according to at least one of the preceding claims, **characterized in that** the at least two substances which simultaneously have a caring effect and support preservation are present in the impregnation agent at a total content of 0.15% to 0.6% by weight, preferably 0.3% to 0.45% by weight, based on the total weight of the impregnation agent.

6. Cleansing product according to at least one of the preceding claims, **characterized in that** the impregnation agent is free of anionic, amphoteric, nonionic and/or cationic surfactants.

7. Cleansing product according to at least one of the preceding claims, **characterized in that** for the impregnation agent the at least one polymeric compound which has a stabilizing effect is selected from the group of homopolymers or copolymers of acrylic acid and/or acrylamide and derivatives thereof, preferably from the group of acrylates/C10-30 alkyl acrylate crosspolymers.

8. Cleansing product according to at least one of the preceding claims, **characterized in that** the at least one polymeric compound which has a stabilizing effect is present in the impregnation agent at a total content of 0.02% to 0.15% by weight, preferably 0.03% to 0.1% by weight, based on the total weight of the impregnation agent.

9. Cleansing product according to at least one of the preceding claims, **characterized in that** for the impregnation agent the at least one polymeric compound which has a film-forming effect is selected from the group of polymers containing vinylpyrrolidone monomers, preferably from the group of copolymers of polyvinylpyrrolidone, further preferably from PVP hexadecene copolymer and/or PVP eicosene copolymer.

10. Cleansing product according to at least one of the preceding claims, **characterized in that** the at least one polymeric compound which has a film-forming effect is present in the impregnation agent at a total content of 0.2% to 1.0% by weight, preferably 0.3% to 0.6% by weight, based on the total weight of the impregnation agent.

11. Cleansing product according to at least one of the preceding claims, **characterized in that** the at least two polymeric compounds, where at least one polymeric compound has a stabilizing effect and at least one polymeric compound has a film-forming effect or where at least one polymeric compound has a stabilizing and film-forming effect and at least one further polymeric compound is present, are present in the impregnation agent at a total content of 0.22% to 1.2% by weight, preferably 0.33% to 0.7% by weight, based on the total weight of the impregnation agent.

12. Cleansing product according to at least one of the preceding claims, **characterized in that** the impregnation agent contains at least one emollient, preferably selected from monoesters of saturated and/or unsaturated, branched and/or unbranched alkanecarboxylic acids having a chain length of 3 to 30 carbon atoms and saturated and/or unsaturated, branched and/or unbranched alcohols having a chain length of 3 to 30 carbon atoms, esters of aromatic carboxylic acids and saturated and/or unsaturated, branched and/or unbranched alcohols having a chain length of 3 to 30 carbon atoms, dialkyl ethers and/or dialkyl carbonates.

13. Cleansing product according to at least one of the preceding claims, **characterized in that** the at least one emollient is present in the impregnation agent at a total content of 0.05% to 5.0% by weight, preferably 0.1% to 3.5% by weight, based on the total weight of the impregnation agent.

14. Cleansing product according to at least one of the preceding claims, **characterized in that** the impregnation agent is free of silicones.

15. Cleansing product according to at least one of the preceding claims, **characterized in that** the wipe consists of a natural-based fibrous material, preferably viscose, cotton, cellulose, jute, hemp, sisal, silk, wool or mixtures thereof.

16. Cleansing product according to at least one of the preceding claims, **characterized in that** the wipe consists of 100% of viscose, preferably with finenesses of 1.0 dtex to 2.0 dtex, further preferably 1.2 dtex to 1.8 dtex.

17. Cleansing product according to at least one of the preceding claims, **characterized in that** the weight ratio of the unimpregnated wipe to the impregnation agent is 1:1 to 1:5, particularly advantageously 1:1.5 to 1:4.5, especially advantageously 2.0:3.5.

## Revendications

1. Agent de nettoyage constitué d'une lingette, **caractérisé en ce que** la lingette est constituée de fibres d'origine naturelle et biodégradables, et d'un agent d'imprégnation, **caractérisé en ce que** l'agent d'imprégnation se présente sous la forme d'une hydrodispersion exempte de silicone et de parabène, c'est-à-dire qui contient moins de 0,001% en poids par rapport au poids total du milieu d'imprégnation, contenant
- du phénoxyéthanol
- au moins deux substances qui, simultanément, ont un effet de soin et soutiennent la conservation et
- au moins deux composés polymères, au moins un composé polymère ayant un effet stabilisant et au moins un composé polymère ayant un effet filmogène ou au moins un composé polymère ayant un effet stabilisant et filmogène et au moins un autre composé polymère étant contenu,
- **caractérisé en ce que** lesdites au moins deux substances qui, simultanément, ont un effet de soin et soutiennent la conservation sont choisies dans le groupe constitué d'éthylhexylglycérol, d'hydroxyacétophénone et de décylèneglycol.

2. Agent de nettoyage selon la revendication 1, **caractérisé en ce que** l'agent d'imprégnation contient de l'eau à une teneur de 80% à 99% en poids, de préférence de 93% à 98, 9% en poids, par rapport au poids total de l'agent d'imprégnation.

3. Agent de nettoyage selon la revendication 1 et/ou 2, **caractérisé en ce que** l'agent d'imprégnation contient du phénoxyéthanol à une teneur de 0,5% à 0,98% en poids, de préférence de 0,8% à 0,95% en poids, par rapport au poids total de l'agent d'imprégnation.

4. Agent de nettoyage selon au moins l'une des revendications précédentes, **caractérisé en ce que** la combinaison d'éthylhexylglycérol et de décylèneglycol est utilisée dans l'agent d'imprégnation en tant que substances qui, simultanément, ont un effet de soin et soutiennent la conservation.

5. Agent de nettoyage selon au moins l'une des revendications précédentes, **caractérisé en ce que** lesdites au moins deux substances qui, simultanément, ont un effet de soin et soutiennent la conservation sont présentes dans l'agent d'imprégnation à une teneur totale de 0,15% à 0,6% en poids, de préférence de 0,3% à 0,45% en poids, par rapport au poids total de l'agent d'imprégnation.

6. Agent de nettoyage selon au moins l'une des revendications précédentes, **caractérisé en ce que** l'agent d'imprégnation est exempt de tensioactifs anioniques, amphotères, non ioniques et/ou cationiques.

7. Agent de nettoyage selon au moins l'une des revendications précédentes, **caractérisé en ce que** pour l'agent d'imprégnation, ledit au moins un composé polymère ayant un effet stabilisant est choisi dans le groupe constitué des homopolymères ou copolymères de l'acide acrylique et/ou de l'acrylamide et de leurs dérivés, de préférence dans le groupe constitué des polymères réticulés d'acrylates/acrylates d'alkyle en C10-30.

8. Agent de nettoyage selon au moins l'une des revendications précédentes, **caractérisé en ce que** l'agent d'imprégnation contient ledit au moins un composé polymère ayant un effet stabilisant à une teneur totale de 0,02% à 0,15% en poids, de préférence de 0,03% à 0,1% en poids, par rapport au poids total de l'agent d'imprégnation.

9. Agent de nettoyage selon au moins l'une des revendications précédentes, **caractérisé en ce que** pour l'agent d'imprégnation, ledit au moins un composé polymère ayant un effet filmogène est choisi dans le groupe des polymères contenant des monomères de vinylpyrrolidone, de préférence dans le groupe des copolymères de polyvinylpyrrolidone, plus préférablement dans le groupe des copolymères de PVP-hexadécène et/ou de PVP-eicosène.

10. Agent de nettoyage selon au moins l'une des revendications précédentes, **caractérisé en ce que** l'agent d'imprégnation contient ledit au moins un composé polymère ayant un effet filmogène à une teneur totale de 0,2% à 1,0% en poids, de préférence de 0,3% à 0,6% en poids, par rapport au poids total de l'agent d'imprégnation.

11. Agent de nettoyage selon au moins l'une des revendications précédentes, **caractérisé en ce que** l'agent d'imprégnation contient lesdits au moins deux composés polymères, au moins un composé polymère ayant un effet stabilisant et au moins un composé polymère ayant un effet filmogène, ou au moins un composé polymère ayant un effet stabilisant et un effet filmogène et au moins un autre composé polymère étant présent, à une teneur totale de 0,22% à 1,2% en poids, de préférence de 0,33% à 0,7% en poids, par rapport au poids total de l'agent d'imprégnation.

12. Agent de nettoyage selon au moins l'une des revendications précédentes, **caractérisé en ce que** l'agent d'imprégnation contient au moins un émollient, de préférence choisi parmi les monoesters d'acides alcanecarboxyliques saturés et/ou insaturés, ramifiés et/ou non ramifiés, d'une longueur de chaîne de 3 à 30 atomes de carbone et d'alcools saturés et/ou insaturés, ramifiés et/ou non ramifiés d'une longueur de chaîne de 3 à 30 atomes de carbone, les esters d'acides carboxyliques aromatiques et d'alcools saturés et/ou insaturés, ramifiés et/ou non ramifiés d'une longueur de chaîne de 3 à 30 atomes de carbone, les dialkyléthers et/ou les carbonates de dialkyle.

13. Agent de nettoyage selon au moins l'une des revendications précédentes, **caractérisé en ce que** l'agent d'imprégnation contient ledit au moins un émollient à une teneur totale de 0,05% à 5,0% en poids, de préférence de 0,1% à 3,5% en poids, par rapport au poids total de l'agent d'imprégnation.

14. Agent de nettoyage selon au moins l'une des revendications précédentes, **caractérisé en ce que** l'agent d'imprégnation est exempt de silicones.

15. Agent de nettoyage selon au moins l'une des revendications précédentes, **caractérisé en ce que** la lingette est constituée d'un matériau fibreux à base naturelle, de préférence de viscose, de coton, de cellulose, de jute, de chanvre, de sisal, de soie, de laine ou leurs mélanges.

16. Agent de nettoyage selon au moins l'une des revendications précédentes, **caractérisé en ce que** la lingette est constituée, à raison de 100%, de viscose, de préférence présentant des finesses de 1,0 dtex à 2,0 dtex, plus préférablement de 1,2 dtex à 1,8 dtex.

17. Agent de nettoyage selon au moins l'une des revendications précédentes, **caractérisé en ce que** le rapport pondéral de la lingette non imprégnée à l'agent d'imprégnation est de 1:1 à 1:5, de manière particulièrement avantageuse de 1:1,5 à 1:4,5, de manière tout particulièrement avantageuse de 2,0:3,5.
